# EUROPEAN PATENT SPECIFICATION

(11) **EP 1 791 603 B1**
(45) Date of publication and mention of the grant of the patent: **09.05.2012**
(21) Application number: 05756647.3
(22) Date of filing: 21.06.2005
(51) Int. Cl.: A61K 8/84, A61Q 15/00

(54) **COSMETIC COMPOSITION COMPRISING AS ANTIPERSPIRANT AGENT A FLOCCULATING WATER-SOLUBLE POLYMER; PROCESS FOR TREATING PERSPIRATION**
KOSMETISCHE ZUSAMMENSETZUNG MIT EINEM FLOCKENDEN WASSERLÖSLICHEN POLYMER ALS ANTIPERSPIRANS; VERFAHREN ZUR BEHANDLUNG VON PERSPIRATION
COMPOSITION COSMETIQUE COMPRENANT UN SOLVANT HYDROSOLUBLE FLOCULANT EN TANT QU'AGENT ANTITRANSPIRATION ET PROCEDE DE TRAITEMENT DE LA TRANSPIRATION

(30) Priority: 18.08.2004 FR 0451867; 28.09.2004 US 613517 P
(43) Date of publication of application: 06.06.2007
(73) Proprietor: L'Oréal, 75008 Paris (FR)
(72) Inventor: LEMOINE, Cyril, 78210 SAINT-CYR L'ECOLE (FR); BEAU, Nathalie, F-95610 Eragny-sur-Oise (FR)
(74) Representative: Miszputen, Laurent
(86) International application number: PCT/EP2005/007370
(87) International publication number: WO 2006/018073

(56) References cited:
- US-A- 5 863 524
- US-A- 5 955 415
- DATABASE WPI Week 198047 Derwent Publications Ltd., London, GB; AN 1980-83626c XP002323616 & JP 55 130906 A (SHISEIDO CO) 11 October 1980 (1980-10-11)

## Description

The invention relates to a cosmetic process for treating the human perspiration which consists in applying to the skin an effective amount of a cosmetic composition comprising at least one aqueous phase and an antiperspirant active agent, characterized in that the said antiperspirant active agent consists of at least one flocculating water-soluble polymer comprising amine groups on the main chain as defined in claim 1 and in that it does not contain any aluminium and/or zirconium antiperspirant salts.

It is well known in cosmetics to use in topical application antiperspirant products containing substances that have the effect of limiting or even suppressing the flow of sweat. These products are generally available in the form of roll-ons, sticks, aerosols or sprays.

Antiperspirant substances generally consist of aluminium salts or aluminium/zirconium salts. Their antiperspirant efficacy is limited when they are used alone. The use of these active agents at high concentrations to obtain good efficacy results in formulation difficulties. Furthermore, these substances have an irritant potential to the skin.

There is thus a need to find novel antiperspirant active agents that can replace aluminium salts and aluminium/zirconium salts, and that are effective and easy to formulate.

Water-insoluble polymers forming an occlusive film on the skin have already been proposed in patent application WO 95/24105 as antiperspirant active agents. It is not necessary to use standard aluminium salts. The occlusive polymers proposed are of the octacrylamide/acrylates copolymer type or of the vinyl acetate/butyl maleate/isobornyl acrylates copolymer type, alone or in combination with a PVP/linear α-olefin polymer, for instance PVP/eicosene.

Water-insoluble film-forming polymers whose main chain is hydrocarbon-based and which comprise pendent hydrophobic quaternary ammonium groups have also been proposed in patent application WO 95/27473 as antiperspirant active agents.

Patent application WO 01/54658 discloses anhydrous compositions containing a cyanoacrylate monomer that reacts with sweat to form in situ by polymerization a film on the skin that blocks the sweat ducts.

However, these occlusive film-forming polymers do not make it possible to obtain fully satisfactory antiperspirant efficacy and still elicit formulation problems.

Moisture-absorbing polymers have been proposed as substitutes for standard astringent salts in antiperspirant anhydrous aerosol compositions in patents US 4 743 440 and US 4 822 596. These moisture-absorbing polymers may especially be water-soluble and chosen especially from ionenes, natural gums (xanthan, agar, carrageenans, guar or gelatin), celluloses (hydroxypropylmethylcellulose, carboxymethylcellulose), polyoxyethylenes, polyvinylpyrrolidones, polycarboxyvinyl polymers or vinyl ether/maleic anhydride copolymers. The ionene-based anhydrous aerosol compositions described in the said document are not satisfactory as regards the antiperspirant efficacy.

In patent application WO 03/030 853, the recommended moisture-absorbing polymers are chosen from grafted starch homopolymers and 2-propenamide-co-propenoic acid sodium salt copolymers.

However, these moisture-absorbing polymers do not make it possible to obtain fully satisfactory antiperspirant efficacy and still elicit formulation problems.

Water-soluble quaternary polymers have been proposed in antiperspirant compositions in the presence of standard aluminium salts to improve their efficacy. This is the case for dimethyldiallylammonium chloride in patent application EP 222 580, which acts as an agent for retaining the antiperspirant salt. This is the case for the water-soluble polymers comprising a Brönsted acid in patent application WO 02/49590, in particular those derived from maleic acid and/or maleic anhydride, which act as co-gelling agent with the antiperspirant salts. This is the case for polyethyleneimines (PEI) in the article Cosmetics & Toiletries Vol. 108 August 1993 pages 73-77, which act as complexing agent.s for the aluminium salts.

Dimethyldiallylammonium chloride/acrylic acid copolymers have been proposed in patent application EP 478 327 as thickeners in aqueous liquid antiperspirant products containing aluminium salts.

In patent US 4 690 817, film-forming polymers of vinyl alcohols containing pendent quaternary amine groups have been proposed in antiperspirant compositions in the presence of standard astringent salts as skin conditioners, forming a moisturizing barrier thereon.

In patent application WO 82/01993, polyethyleneimines have been used as odour absorbers in particular of fatty acids, aldehydes or ketones and more particularly in alcohol-based deodorant products in spray or roll-on form. The said document does not describe or suggest any activity of these polymers on the flow of sweat. The said document does not describe or deal with the moisture problems of perspiration.

**In patent** US5863524 polyalkyleneamines have been used as clarifying agents for deodorant formulations based on alkaline metal bicarbonate salt.

The Applicant has discovered, surprisingly, that flocculating water-soluble cationic polymers comprising amine groups on the main chain constitute by themselves excellent antiperspirant agents and can be easily formulated in numerous products for treating perspiration, without, it being necessary to use standard astringent salts.

A subject of the present invention is also a cosmetic process for treating perspiration, which consists in applying to the surface of the skin an effective amount of such a composition as defined in claim 1.

A subject of the present invention is also the use of a flocculating water-soluble polymer comprising amine groups on the main chain as defined in claim 1 as antiperspirant active agent in a cosmetic composition, and in particular in a cosmetic composition comprising an aqueous phase.

A subject of the present invention is also the use of a flocculating water-soluble polymer comprising amine groups on the main chain as an antiperspirant active agent in a composition not containing any aluminium and/or zirconium

The term "antiperspirant agent" means any substance that has the effect of reducing or limiting the flow of sweat.

The term "water-soluble polymer" means polymers which, when introduced into an aqueous phase at 25°C, at a mass concentration equal to 1%, make it possible to obtain a macroscopically homogeneous and transparent solution, i.e. a solution that has a minimum light transmittance value, at a wavelength equal to 500 nm, through a sample 1 cm thick, of at least 80% and preferably of at least 90%.

The term "flocculant" means any substance capable of destabilizing a colloidal suspension indistinctly via a flocculation or coagulation mechanism. The term "destabilization of colloids" means the formation of aggregates that make the suspension unstable. Since the terms flocculation and coagulation are generally interchangeable and equivalent, we will use the term "flocculation" in the invention to refer to either mechanism. The definitions of these mechanisms are given in Volume 10 of "Encyclopedia of Chemical Technology"; Kirk-Othmer; 3rd edition.

From an experimental point of view, according to the invention, a flocculant will be considered as effective in antiperspirant terms if it reduces by at least 10% and better still 20% the transmittance measured at a wavelength of 700 nm of a solution of natural sweat filtered through a 200 micron filter.

The term "composition not containing any aluminium and/or zirconium antiperspirant salts" means any composition containing not more than 1% by weight of aluminium and/or zirconium antiperspirant salt.

The term "composition comprising at least one aqueous phase" means a cosmetic composition comprising at least 1% water and preferably at least 10% water.

Flocculating water-soluble polymers comprise on the main chain amine functions partially or totally ionized in quaternary amine form.

According to the invention, the term "polymers with amines partially ionised in quaternary amine form" means any polymer for which at least one amine of the main chain is in quaternary amine form between pH 4 and pH 8.

The flocculating water-soluble polymers are chosen from: polyalkyleneamines as defined in claim 1.

### - Polyalkyleneimines

The polymers of the invention may be derived from the polymerization reaction using an alkyleneimine (preferably ethyleneimine). Such polymers are commonly known as polyalkyleneimines.

Such polymers may also be derived from the condensation reaction of an alkyldlamine with a dihaloalkyl derivative.

The polyalkyleneimines used according to the invention are polymers containing from 8 to 20 000 repeating units.

The polyalkyleneimines used in accordance with the invention generally have a weight-average molecular weight ranging from 300 to 100 000, preferably from 350 to 50 000, more particularly from 400 to 10 000 and preferably from 500 to 2000. The molecular weights may be determined by quasi-elastic light scattering.

Polyalkyleneimines are especially described in the book "Polymer Science Dictionary" 2nd edition, Mark Alger, Chapman & Hall, 1997, and also in the document "The polymerization of olefin imines" by Jones, in P.H. Plesch ed., The chemistry of cationic polymerization, New York, MacMillan (1963) pages 521-534.

The polyalkyleneamines of the invention correspond to the structure: 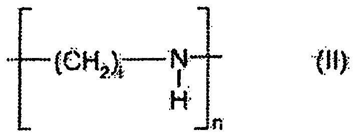 in wich i is an integer ranging from 2 to 20. approximately and preferably less than 6, and n is an integer ranging from 10 to 10⁶ and preferably from 10 to 10⁴.

One particular and preferred form corresponds to the ethyleneimine homopolymers having the structure: 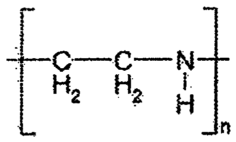 n is an integer ranging from 10 to 10⁶ and preferably from 10 to 10⁴.

Examples than may be mentioned include the following polyethyleneimines (INCI name; PEI) : PEI-7, PEI-10, PEI-15, PEI-30, PEI-35, PEI-45, PEI-250, PEI-275, PEI-700, PEI-1000, PEI-1400, PEI-1500, PEI-1750, PEI-2500, PEI-14000.

These polyethyleneimines are sold under the following trade names by the company BASF:
- Lupasol FG (PEI-10): the primary/secondary/tertiary amine ratios are: 42/35/23, the molar mass being 800 g/mol,
- Lupasol G-35 (PEI-35) : the primary/secondary/tertiary amine ratios are: 38/36/26, the molar mass being 2000 g/mol.
- Lupasol Water Free (PEI-250): the primary/secondary/- tertiary amine ratios are: 34/40/26, the molar mass being 25 000 g/mol,
- Lupasol P and Lupasol PS (PEI-1500): the primary/- secondary/tertiary amine ratios are: 35/38/27, the molar mass being 750 000 g/mol.

These polyethyleneimines are sold under the trade name Epomin by the company Aceto Corporation:
- Epomin SP-006 (PEI-15); Epomin SP-012 (PEI-30); Epomin SP 0.15 (PEI-45), Epomin P1000 (PEI-1750) ; Epomin P1010.

The flocculating water-soluble polymers used as antiperspirant active agents are preferably present in the compositions according to the invention in amounts ranging from 0.1% to 50% and more preferably from 1% to 20% by weight relative to the total weight of the composition.

According to one particular form of the invention, the antiperspirant compositions comprise at least 5% of flocculating water-soluble polymer as described above.

The compositions according to the invention intended for cosmetic use may be in the form of lotions, creams or fluid gels distributed as an aerosol spray, in a pump-dispenser bottle or as a roll-on, in the form of thick creams distributed in tubes or a grille; in the form of wands (sticks), and may contain in this regard the ingredients generally used in products of this type and well known to those skilled in the art.

The compositions according to the present invention intended for cosmetic use may comprise at least one aqueous phase. They are especially formulated as aqueous lotions or as water-in-oil, oil-in-water emulsions, or as multiple emulsions (oil-in-water-in-oil or water-in-oil-in-water triple emulsions (such emulsions are known and described, for example, by C. Fox in "Cosmetics and Toiletries", November 1986, Vol. 101, pages 101-112)).

The aqueous phase of the said compositions contains water and generally other water-soluble or water-miscible solvents. The water-soluble or water-miscible solvents include short-chain monoalcohols, for example of C₁-C₄, for instance ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Propylene glycol and glycerol will more particularly be used.

According to one particular form of the invention, the antiperspirant compositions comprise at least 5% of flocculating polymer as described above.

The compositions according to the invention preferably comprise at least one water-immiscible organic liquid phase.

This phase generally comprises one or more hydrophobic compounds that render the said phase water-immiscible. The said phase is liquid (in the absence of a structuring agent) at room temperature (20-25°C). Preferentially the water-immiscible organic liquid phase in accordance with the invention generally consists of an oil or a mixture of oils and comprises at least 80% of compounds with a vapour pressure not exceeding 4 kPa (30 mmHg) at 25°C.

The water-immiscible organic liquid phase preferably contains one or more volatile or non-volatile, silicone-based or hydrocarbon-based emollient oils. These emollient oils are especially described in patents US 4 822 596 and US 4 904 463.

Volatile silicones are defined, in a known manner, as being compounds that are volatile at room temperature. Mention may be made among these compounds of cyclic and linear volatile silicones of the dimethylsiloxane type whose chains comprise from 3 to 9 silicone-based residues. Cyclomethicones D₄, D₅ or D₆ are preferably chosen.

Non-volatile silicones are defined, in a known manner, as being compounds with a low vapour pressure at room temperature. The following are included among these compounds: polyalkylsiloxanes, in particular linear polyalkylsiloxanes, for instance the linear polydimethylsiloxanes, or dimethicones, sold by the company Dow Corning under the name "Dow Corning 245 Fluid"; polyalkylarylsiloxanes, for instance the polymethylphenylsiloxanes sold by the company Dow Corning under the name "Dow Corning 556 Fluid"; copolymers of polyether and siloxane, for instance dimethicone copolyols.

Among the non-volatile emollient oils that may be used in the present invention, examples that may be mentioned include: hydrocarbon-based derivatives, mineral oils, fatty alcohols, esters of C₃-C₁₈ alcohols with C₃-C₁₈ acids, esters of benzoic acid with C₁₂-C₁₈ alcohols and mixtures thereof, C₂-C₆ polyols preferably chosen from glycerol, propylene glycol or sorbitol, polyalkylene glycol polymers.

The emollient oils are preferably present in amounts ranging from 1% to 50% by weight and more preferably from 5% to 40% by weight relative to the total weight of the composition.

The cosmetic composition according to the invention may contain one or more additional deodorant active agents, for instance bacteriostatic agents or bactericidal agents such as 2,4,4'-trichloro-2'-hydroxydiphenyl ether (Triclosan), 2,4-dichloro-2'-hydroxydiphenyl ether, 3',4',5'-trichlorosalicyl-anilide, 1-(3',4'-dichlorophenyl)-3-(4'-chlorophenyl)-urea (Triclocarban) or 3,7,11-trimethyldodeca-2,5,10-trienol (Farnesol); quaternary ammonium salts, for instance cetyltrimethylammonium salts or cetylpyridinium salts; chlorhexidine and salts; diglyceryl monocaprate, diglyceryl monolaurate or glyceryl monolaurate; polyhexamethylene biguanide salts.

In order to improve the antiperspirant efficacy of the composition, one or more water-soluble anionic polymers comprising a Brönsted acid, in particular those derived from maleic acid and/or from maleic anhydride, which are described in patent application WO 02/49590, may also be used.

In order to improve the homogeneity of the product, it is possible also to use one or more suspenders preferably chosen from hydrophobic-modified montmorillonite clays, for instance hydrophobic-modified bentonites or hectorites. Examples that may be mentioned include the product stearalkonium bentonite (CTFA name) (product of reaction of bentonite and the quaternary ammonium stearalkonium chloride), such as the commercial product sold under the name Tixogel MP 250 by the company Süd Chemie Rheologicals, United Catalysts Inc. or the product disteardimonium hectorite (CTFA name) (product of reaction of hectorite and of distearyldimonium chloride) sold under the name Bentone 38 or Bentone Gel by the company Elementis Specialities.

The suspenders are preferably present in amounts ranging from 0.1% to 5% by weight and more preferably from 0.2% to 2% by weight relative to the total weight of the composition.

The compositions according to the invention may also contain at least one organic powder.

Among the fillers that may be used according to the invention, mention may be made of organic powders. In the present patent application, the term "organic powder" means any solid that is insoluble in the medium at room temperature (25°C).

As organic powders that may be used in the composition of the invention, examples that may be mentioned include polyamide particles and especially those sold under the name Orgasol by the company Atochem; polyethylene powders; microspheres based on acrylic copolymers, such as those made of ethylene glycol dimethacrylate/lauryl methacrylate copolymer, sold by the company Dow Corning under the name Polytrap; polymethyl methacrylate microspheres, sold under the name Microsphere M-100 by the company Matsumoto or under the name Covabead LH85 by the company Wackherr; ethylene-acrylate copolymer powders, for instance those sold under the name Flobeads by the company Sumitomo Seika Chemicals; expanded powders such as hollow microspheres and especially microspheres formed from a terpolymer of vinylidene chloride, of acrylonitrile and of methacrylate and sold under the name Expancel by the company Kemanord Plast under the references 551 DE 12 (particle size of about 12 µm and density of 40 kg/m³), 551 DE 20 (particle size of about 30 µm and a density of 65 kg/m³) and 551 DE 50 (particle size of about 40 µm), or the microspheres sold under the name Micropearl F 80 ED by the company Matsumoto; powders of natural organic materials such as starch powders, especially of corn starch, wheat starch or rice starch, which may or may not be crosslinked, such as the starch powder crosslinked with octenylsuccinate anhydride, sold under the name Dry-Flo by the company National Starch; silicone resin microbeads such as those sold under the name Tospearl by the company Toshiba Silicone, especially Tospearl 240; amino acid powders such as the lauroyllysine powder sold under the name Amihope LL-11 by the company Ajinomoto; particles of wax microdispersion, which preferably have mean sizes of less than 1 µm and especially ranging from 0.02 µm to 1 µm, and which consist essentially of a wax or a mixture of waxes, such as the products sold under the name Aquacer by the company Byk Cera, and especially: Aquacer 520 (mixture of synthetic and natural waxes), Aquacer 514 or 513 (polyethylene wax), Aquacer 511 (polymer wax), or such as the products sold under the name Jonwax 120 by the company Johnson Polymer (mixture of polyethylene wax and paraffin wax) and under the name Ceraflour 961 by the company Byk Cera (micronized modified polyethylene wax); and mixtures thereof. The organic powder(s) may be present, for example, in an amount

The cosmetic composition according to the invention may also comprise cosmetic adjuvants chosen from waxes, softeners, antioxidants, opacifiers, stabilizers, moisturizers, vitamins, fragrances, bactericides, preserving agents, polymers, fragrances, thickeners, propellants or any other ingredient usually used in cosmetics for this type of application.

Needless to say, a person skilled in the art will take care to select this or these optional additional compound(s) such that the advantageous properties intrinsically associated with the cosmetic composition in accordance with the invention are not, or are not substantially, adversely affected by the envisaged addition(s).

The waxes may be chosen from animal, fossil, plant, mineral and synthetic waxes. Mention may be made especially of beeswaxes, carnauba wax, candelilla wax, sugar cane wax, Japan wax, ozokerites, montan wax, microcrystalline waxes, paraffins and silicone waxes and resins.

The thickeners, which are preferably nonionic, may be chosen from modified or unmodified guar gums and celluloses, such as hydroxypropyl guar gum or cetylhydroxyethylcellulose, silicas, for instance Bentone Gel MIO sold by the company NL Industries, or Veegum Ultra sold by the company Polyplastic.

The amounts of these various constituents that may be present in the cosmetic composition according to the invention are those conventionally used in deodorant compositions.

The compositions according to the invention may also contain one or more other agents for structuring or gelling the water-immiscible organic liquid phase of the composition, such as linear solid fatty alcohols and/or waxes; fatty acids or salts thereof (stearic acid, sodium stearate or 12-hydroxystearic acid; dibenzylidene alditols (DBS); lanosterol, N-acylamino acid derivatives; di- or tricarboxylic acid derivatives, for instance alkyl-N,N'-dialkylsuccin-amides (i.e.: dodecyl-N,N'-dibutylsuccinamide); elastomeric polyorganosiloxanes such as those described in patent application WO 97/44010.

The composition according to the invention may also be pressurized and may be packaged in an aerosol device.

A subject of the present invention is an aerosol device consisting of:
(A) a container comprising a deodorant composition as defined above,
(B) at least one propellant and a means for distributing the said aerosol composition.

The propellants generally used in products of this type, which are well known to those skilled in the art, are, for example, dimethyl ether (DME); volatile hydrocarbons such as n-butane, propane or isobutane, and mixtures thereof, optionally with at least one chlorohydrocarbon and/or fluorohydrocarbon; among the latter, mention may be made of the compounds sold by the company Dupont de Nemours under the names Freon^{®} and Dymel^{®}, and in particular monofluorotrichloro-methane, difluorodichloromethane, tetrafluorodichloro-ethane and 1,1-difluoroethane sold especially under the trade name Dymel 152 A by the company Dupont. Carbon dioxide, nitrous oxide, nitrogen or compressed air may also be used as propellant.

The composition containing the deodorant active agent(s) and the propellant(s) may be in the same compartment or in different compartments in the aerosol container. According to the invention, the concentration of propellant generally ranges from 5% to 95% by pressurized weight and more preferably from 50% to 85% by weight relative to the total weight of the pressurized composition.

The distribution means, which forms a part of the aerosol device, generally consists of a distribution valve controlled by a distribution head, itself comprising a nozzle via which the aerosol composition is vaporized. The container containing the pressurized composition may be opaque or transparent. It may be made of glass, of polymeric material or of metal, optionally coated with a layer of protective varnish.

The examples that follow serve to illustrate the present invention.

### Example 1: Antiperspirant vaporizer (PIT emulsion)

| **Ingredients** | **Amounts (g)** |
|---|---|
| PEI 10 - Lupasol FG | 15 |
| Cetearyl isononanoate (and) cetearyl alcohol (and) Cetrareth-20 (and) glycerol (and) glyceryl stearate (and) Ceteareth-12 (and) cetyl palmitate (Emulgade CM) | 15 |
| Water | 70 |
| | 100 |

### Procedure:

The polyalkyleneimine is dissolved in water and the emulsifying agent is added to the mixture with moderate stirring.

## Claims

1. Cosmetic process for treating human perspiration, which consists in applying to the surface of the skin an effective amount of a cosmetic composition comprising at least one aqueous phase and at least one antiperspirant active agent, **characterized in that** the said antiperspirant active agent consists of at least one flocculating water-soluble polymer comprising amine groups on the main chain chosen from polyalkyleneamines and **in that** it does not contain any aluminium and/or zirconium antiperspirant salts ; the said polyalkyleneamines being selected from polyalkyleneamines of structure : 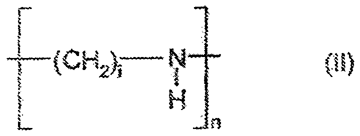 is which i is an integer ranging from 2 to 20 and preferably less than 6, and a is an integer ranging from 10 to 10⁵ and preferably from 10 to 10⁴.

2. **Process** according to Claim 1, in which the polyalkyleneamines contain from 5 to 20 000 repeating units.

3. **Process** according to Claim 1 or 2, in which the polyalkyleneimines comprise at least 5% of tertiary amines, advantageously at least 10% and even more preferably at least 20% of tertiary amine functions.

4. **Process** according to any one of Claims 1 to 3, in which the polyalkyleneimines have a weight-average molecular weight ranging from 300 to 100 000, preferably from 350 to 50 000, more particularly from 400 to 10 000 and preferably from 500 to 2000.

5. **Process** according to Claim 1, in which the polyalkyleneimines are polyethyleneimines of structure: 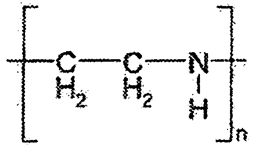 in which n is an integer ranging from 10 two 10⁵ and preferably from 10 to 10⁴.

6. **Process** according to any one of Claims 1 to 5, in which the flocculating water-soluble polymer is present in amounts of greater than 5% by weight.

7. **Process** according to any one of Claims 1 to 6, **characterised in that** the composition is in the form of a lotion, a cream or a fluid get distributed as an aerosol spray, in a pump-dispenser bottle or as a roll-on; in the form of a cream or gel distributed in a tube or grille; in the form of a wand (stick).

8. **Process** according to anyone of Claims 1 to 7, **characterized in that the composition** also comprises one or more additional deodorant active agents or

9. Use of a flocculating water-soluble polymer as defined in any one of Claims **1 to 5** as an: antiperspirant active agent in a cosmetic composition.

10. Use according to Claim **9,** in which the cosmetic composition comprises at least one aqueous phase.

11. Use according to Claim **9** or **10,** in which the cosmetic composition does not contain any aluminium and/or zirconium antiperspirant salts

## Patentansprüche

1. Kosmetisches Verfahren zur Behandlung von menschlicher Schweißabsonderung, das darin besteht, dass man auf die Oberfläche der Haut eine wirksame Menge einer kosmetischen Zusammensetzung, die mindestens eine wässrige Phase und mindestens ein schweißhemmendes Mittel umfasst, aufbringt, **dadurch gekennzeichnet, dass** das schweißhemmende Mittel aus mindestens einem unter Polyalkylenaminen ausgewählten ausflockend wirkenden wasserlöslichen Polymer mit Amingruppen an der Hauptkette besteht und keine schweißhemmenden Aluminium- und/oder Zirconiumsalze enthält; wobei die Polyalkylenamine unter Polyalkyleniminen der Struktur: 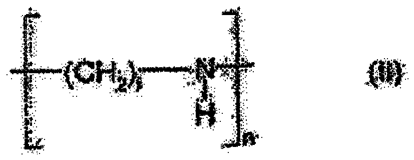 worin i für eine ganze Zahl im Bereich von 2 bis 20 und vorzugsweise weniger als 6 steht und n für eine ganze Zahl im Bereich von 10 bis 10⁶ und vorzugsweise von 10 bis 10⁴ steht, ausgewählt werden.

2. Verfahren nach Anspruch 1, bei dem die Polyalkylenamine 6 bis 20.000 Wiederholungseinheiten enthalten.

3. Verfahren nach Anspruch 1 oder 2, bei dem die Polyalkylenimine mindestens 5% tertiäre Amine, vorteilhafterweise mindestens 10% und noch weiter bevorzugt mindestens 20% tertiäre Aminfunktionen umfassen.

4. Verfahren nach einem der Ansprüche 1 bis 3, bei dem die Polyalkylenimine ein gewichtsmittleres Molekulargewicht im Bereich von 300 bis 100.000, vorzugsweise von 350 bis 50.000, spezieller von 400 bis 10.000 und vorzugsweise von 500 bis 2000 aufweisen.

5. Verfahren nach Anspruch 1, bei dem es sich bei den Polyalkyleniminen um Polyethylenimine der Struktur: 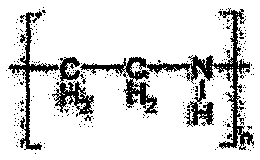 worin n für eine ganze Zahl im Bereich von 10 bis 10⁶ und vorzugsweise von 10 bis 10⁴ steht, handelt.

6. Verfahren nach einem der Ansprüche 1 bis 5, bei dem das ausflockend wirkende wasserlösliche Polymer in Mengen von mehr als 5 Gew.-% vorliegt.

7. Verfahren nach einem der Ansprüche 1 bis 6, **dadurch gekennzeichnet, dass** die Zusammensetzung in Form einer Lotion, einer Creme oder eines fluiden Gels, die bzw. das als Aerosolspray, in einer Pumpspenderflasche oder als Roller verteilt wird; in Form einer Creme oder eines Gels, die bzw. das aus einer Tube oder über ein Gitter verteilt wird; oder in Form eines Stifts (Sticks) vorliegt..

8. Verfahren nach einem der Ansprüche 1 bis 7, **dadurch gekennzeichnet, dass** die Zusammensetzung auch ein oder mehrere zusätzliche Desodorantien oder ein oder mehrere Bakteriostatika Bakterizide umfasst.

9. Verwendung eines ausflockend wirkenden wasser-löslichen Polymers gemäß einem der Ansprüche 1 bis 5 als schweißhemmendes Mittel in einer kosmetischen Zusammensetzung.

10. Verwendung nach Anspruch 9, wobei die kosmetische Zusammensetzung mindestens eine wässrige Phase umfasst.

11. Verwendung nach Anspruch 9 oder 10, wobei die kosmetische Zusammensetzung keine schweißhemmenden Aluminium- und/oder Zirconiumsalze enthält.

## Revendications

1. Procédé cosmétique de traitement de la transpiration humaine, qui consiste en l'application à la surface de la peau d'une quantité efficace d'une composition cosmétique comprenant au moins une phase aqueuse et au moins un agent actif anti-transpirant, **caractérisé en ce que** ledit agent actif anti-transpirant est constitué d'au moins un polymère hydrosoluble floculant comprenant des groupements amine sur la chaîne principale choisis parmi des polyalkylèneamines et **en ce qu'**il ne contient aucun sel anti-transpirant à base d'aluminium et/ou de zirconium ; lesdites polyalkylèneamines étant choisies parmi les polyalkylèneimines de structure : 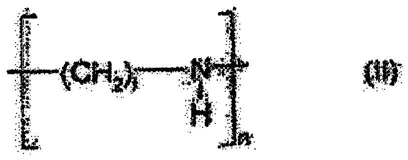 dans laquelle i est un nombre entier allant de 2 à 20 et préférablement inférieur à 6, et n est un nombre entier allant de 10 à 10⁶ et préférablement de 10 à 10⁴.

2. Procédé selon la revendication 1, dans lequel les polyalkylèneamines contiennent de 6 à 20 000 motifs répétitifs.

3. Procécé selon la revendication 1 ou 2, dans lequel les polyalkylèneimines comprennent au moins 5% d'amines tertiaires, de manière avantageuse au moins 10% et encore plus préférablement au moins 20% de fonctions amine tertiaire.

4. Procédé selon l'une quelconque des revendications 1 à 3, dans lequel les polyalkylèneimines ont un poids moléculaire moyen en poids allant de 300 à 100 000, préférablement de 350 à 50 000, plus préférablement de 400 à 10 000 et préférablement de 500 à 2000.

5. Procédé selon la revendication 1, dans lequel les polyalkylèneimines sont des polyalkylèneimines de structure : 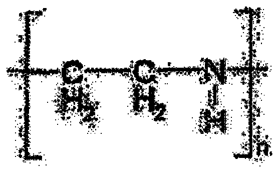 dans laquelle n est un nombre entier allant de 10 à 10⁶ et préférablement de 10 à 10⁴.

6. Procédé selon, l'une quelconque des revendications 1 à 5, dans lequel le polymère hydrosoluble floculant est présent en dans quantitiés supérieures à 5% en poids.

7. Procédé selon l'une quelconque des revendications 1 à 6, **caractérisé en ce que** la composition se trouve sous la forme d'une lotion, d'une crème, ou d'un gel fluide distribué sous forme de spray aérosol, dans un flacon à bouchon gicleur ou sous forme de roll-on ; sous la forme d'une crème ou d'un gel distribué(e) dans un tube ou une grille ; sous la forme d'un bâton (stick).

8. Procédé selon l'une quelconque des revendications 1 à 7, **caractérisé en ce que** la composition comprend également un ou plusieurs agents actifs déodorants supplémentaires ou un ou plusieurs agents bactériostatiques ou bactéricides.

9. Utilisation d'un polymère hydrosoluble floculant tel que défini selon l'une quelconque des revendications 1 à 5, comme agent actif anti-transpirant dans une composition cosmétique.

10. Utilisation selon la revendication 9, dans laquelle la composition cosmétique comprend au moins une phase aqueuse.

11. Utilisation selon la revendication 9 ou 10, dans laquelle la composition cosmétique ne contient aucun sel anti-transpirant à base d'aluminium et/ou de zirconium.
